# EUROPÄISCHE PATENTSCHRIFT

(11) **EP 2 121 559 B1**
(45) Veröffentlichungstag und Bekanntmachung des Hinweises auf die Patenterteilung: **26.08.2015**
(21) Anmeldenummer: 08708849.8
(22) Anmeldetag: 11.02.2008
(51) Int. Cl.: C07C 67/08, C07C 69/54, C07D 207/273

(54) **KATALYTISCHES VERFAHREN ZUR HERSTELLUNG VON (METH)ACRYLSÄUREESTERN VON N-HYDROXYALKYLIERTEN LACTAMEN**
CATALYTIC METHOD FOR PRODUCING (METH)ACRYLIC ACID ESTERS OF N-HYDROXYALKYLATED LACTAMS
PROCÉDÉ CATALYTIQUE POUR LA PRÉPARATION D'ESTERS D'ACIDE (MÉTH)ACRYLIQUE À PARTIR DE LACTAMES N-HYDROXYALKYLÉS

(30) Priorität: 15.02.2007 EP 07102484
(43) Veröffentlichungstag der Anmeldung: 25.11.2009
(73) Patentinhaber: BASF SE, 67056 Ludwigshafen (DE)
(72) Erfinder: BERGMANN, Hermann, Singapore 098652 (SG); HÖFER, Frank, 67098 Bad Dürkheim (DE); ANGEL, Maximilian, 67105 Schifferstadt (DE)
(86) Internationale Anmeldenummer: PCT/EP2008/051586
(87) Internationale Veröffentlichungsnummer: WO 2008/098887

(56) Entgegenhaltungen:
- WO-A-03/006568
- WO-A-2006/012980
- GB-A- 930 668

## Beschreibung

Die vorliegende Erfindung betrifft ein Verfahren zur katalytischen Herstellung von (Meth)acrylsäureestern von N-hydroxyalkylierten Lactamen.

Unter (Meth)acrylsäure im Sinne der vorliegenden Erfindung werden Acrylsäure und/oder Methacrylsäure verstanden, unter (Meth)acrylsäureester Acrylsäureester und/oder Methacrylsäureester.

Die Herstellung von (Meth)acrylsäureestern erfolgt zumeist durch katalytische Veresterung von (Meth)acrylsäure oder Umesterung von anderen (Meth)acrylsäureestern mit Alkoholen. Dabei werden häufig starke Säuren oder Basen eingesetzt, so dass sich säure- oder baseempfindliche (Meth)acrylsäureester durch eine Ver- oder Umesterung auf diesem Wege in der Regel nicht gezielt herstellen lassen.

(Meth)acrylsäureester von N-hydroxyalkylierten Lactamen sind bekannt.

WO 03/006568 A1 beschreibt die saure Vesterung von Acrylsäure mit Hydroxyethylpyrrolidon mit p-Toluolsulfonsäure als Katalysator. Die Ausbeute beträgt jedoch lediglich 71 %.

Aus der bisher unveröffentlichten deutschen Anmeldung mit dem Aktenzeichen DE 10 2005 052 931.3 ist ein katalytisches Verfahren zur Herstellung von (Meth)acrylsäureestern von N-hydroxyalkylierten Lactamen bekannt, bei dem die Ver- oder Umesterung in Gegenwart eines heterogenen anorganischen Salzes durchgeführt wird.

Aus DE 1 595 233 und GB 930 668 ist die Umesterung von N-Hydroxyalkyl-Lactamen mit (Meth)acrylsäureestern in Gegenwart von Alkalimetall- und ammoniumalkoholaten sowie Titantetraalkoholaten bekannt. Nachteilig an diesen Verfahren ist, dass die im Produkt verbleibenden Spuren der Katalysatoren eine eventuell nachfolgende Polymerisation beeinflussen, und daher aufwendig aus dem Produkt entfernt werden müssen. Eine solche Entfernung wird zumeist mittels einer wässrigen Wäsche durchgeführt, so dass das Produkt anschließend getrocknet werden muss.

Aus GB 930 668 wird ebenfalls die Herstellung von (Meth)acrylsäureestern von N-hydroxyalkylierten Lactamen durch Reaktion von N-Hydroxyalkyl-Lactamen mit (Meth)acrylsäurechlorid beschrieben. Die Verwendung von (Meth)acrylsäurechlorid bei den beschriebenen Umsetzungen führt jedoch zur Salzbildung und wegen dessen hoher Reaktivität zu unselektiven Reaktionen, wie beispielsweise Michael-Additionen.

WO 2006/012980 A offenbart ein Verfahren zur katalytischen Herstellung von (Meth)acrylaten von N-hydroxylierten Amiden unter Verwendung von anorganischen Salzen und Enzymen als Katalysatoren.

Aufgabe der vorliegenden Erfindung war es daher, ein alternatives Verfahren zur Verfügung zu stellen, mit dem (Meth)acrylsäureester von N-hydroxyalkylierten Lactamen in hohen Umsätzen und hohen Reinheiten aus einfachen Edukten herstellbar sind. Die Synthese sollte unter milden Bedingungen ablaufen, so dass Produkte mit einer niedrigen Farbzahl und hoher Reinheit resultieren. Insbesondere soll die Durchführung des Verfahrens industriell machbar sein.

Die Aufgabe wurde gelöst durch ein Verfahren zur Herstellung von (Meth)acrylsäureestern (F) von N-hydroxyalkylierten Lactamen, in dem man ringförmige N-hydroxyalkylierte Lactame (L), 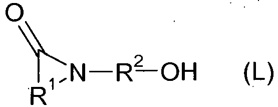 worin
R¹ C₁-C₅-Alkylen oder durch ein- oder mehrere Sauerstoff- und/oder Schwefelatome und/oder ein oder mehrere substituierte oder unsubstituierte Iminogruppen und/oder durch eine oder mehrere Cycloalkyl-, -(CO)-, -O(CO)O-, -(NH)(CO)O-, -O(CO)(NH)-, -O(CO)- oder -(CO)O-Gruppen unterbrochenes C₂-C₂₀-Alkylen, wobei die genannten Reste jeweils durch Aryl, Alkyl, Aryloxy, Alkyloxy, Heteroatome und/oder Heterocyclen substituiert sein können
mit der Maßgabe, dass R¹ kein anderes Atom als ein Kohlenstoffatom direkt benachbart zur Lactam-Carbonylgruppe aufweisen darf,
R² C₁-C₂₀-Alkylen, C₅-C₁₂-Cycloalkylen, C₆-C₁₂-Arylen oder durch ein- oder mehrere Sauerstoff- und/oder Schwefelatome und/oder ein oder mehrere substituierte oder unsubstituierte Iminogruppen und/oder durch eine oder mehrere Cycloalkyl-, -(CO)-, -O(CO)O-, -(NH)(CO)O-, -O(CO)(NH)-, -O(CO)- oder -(CO)O-Gruppen unterbrochenes C₂-C₂₀-Alkylen, wobei die genannten Reste jeweils durch Aryl, Alkyl, Aryloxy, Alkyloxy, Heteroatome und/oder Heterocyclen substituiert sein können, oder
R²-OH für eine Gruppe der Formel-[Xᵢ]ₖ-H,
k für eine Zahl von 1 bis 50 und
Xᵢ für jedes i = 1 bis k unabhängig voneinander ausgewählt sein kann aus der Gruppe -CH₂-CH₂-O-, -CH₂-CH(CH₃)-O-, -CH(CH₃)-CH₂-O-, -CH₂-C(CH₃)₂-O-, -C(CH₃)₂-CH₂-O-, -CH₂-CH₂-CH₂-O-, -CH₂-CH₂-CH₂-CH₂-O-, -CH₂-CHVin-O-, -CHVin-CH₂-O-, -CH₂-CHPh-O- und -CHPh-CH₂-O-, worin Ph für Phenyl und Vin für Vinyl steht,
bedeuten,
in Gegenwart mindestens mindestens eines Katalysators (K) ausgewählt aus der Gruppe bestehend aus Alkalimetallhydroxiden, Erdalkalimetallhydroxiden und Metallacetylacetonaten mit (Meth)acrylsäure (S) verestert oder mit mindestens einem (Meth)acrylsäureester (D) umestert.

Im Folgenden werden die Edukte (Meth)acrylsäure (S) und (Meth)acrylsäureester (D) auch gemeinsam unter dem Begriff (Meth)acrylverbindung (B) zusammengefasst.

Mit Hilfe des erfindungsgemäßen Verfahrens ist die Herstellung von (Meth)acrylaten von N-hydroxyalkylierten Lactamen mit mindestens einem der folgenden Vorteile möglich:
- hohe Ausbeute,
- milde Reaktionsbedingungen,
- gute Farbzahlen und
- keine Waschschritte zur Aufreinigung des Reaktionsgemischs erforderlich.

In den obigen Definitionen bedeuten
gegebenenfalls durch Aryl, Alkyl, Aryloxy, Alkyloxy, Heteroatome und/oder Heterocyclen substituiertes C₁-C₂₀-Alkylen beispielsweise Methylen, 1,2-Ethylen, 1,2-Propylen, 1,3-Propylen, 1,4-Butylen, 1,6-Hexylen, 2-Methyl-1,3-propylen, 2-Ethyl-1,3-propylen, 2,2-Dimethyl-1,3-propylen, 2,2-Dimethyl-1,4-butylen,
gegebenenfalls durch Aryl, Alkyl, Aryloxy, Alkyloxy, Heteroatome und/oder Heterocyclen substituiertes C₅-C₁₂-Cycloalkylen beispielsweise Cyclopropylen, Cyclopentylen, Cyclohexylen, Cyclooctylen, Cyclododecylen,
gegebenenfalls durch Aryl, Alkyl, Aryloxy, Alkyloxy, Heteroatome und/oder Heterocyclen substituiertes, durch ein- oder mehrere Sauerstoff- und/oder Schwefelatome und/oder ein oder mehrere substituierte oder unsubstituierte Iminogruppen und/oder durch eine oder mehrere Cycloalkyl-, -(CO)-, -O(CO)O-, -(NH)(CO)O-, -O(CO)(NH)-, -O(CO)- oder -(CO)O-Gruppen unterbrochenes C₁-C₂₀-Alkylen beispielsweise 1-Oxa-1,3-propylen, 1,4-Dioxa-1,6-hexylen, 1,4,7-Trioxa-1,9-nonylen, 1-Oxa-1,4-butylen, 1,5-Dioxa-1,8-octylen, 1-Oxa-1,5-pentylen, 1-Oxa-1,7-heptylen, 1,6-Dioxa-1,10-decylen, 1-Oxa-3-methyl-1,3-propylen, 1-Oxa-3-methyl-1,4-butylen, 1-Oxa-3,3-dimethyl-1,4-butylen, 1-Oxa-3,3-dimethyl-1,5-pentylen, 1,4-Dioxa-3,6-dimethyl-1,6-hexylen, 1-Oxa-2-methyl-1,3-propylen, 1,4-Dioxa-2,5-dimethyl-1,6-hexylen, 1-Oxa-1,5-pent-3-enylen, 1-Oxa-1,5-pent-3-inylen, 1,1-, 1,2-, 1,3- oder 1,4-Cyclohexylen, 1,2- oder 1,3-Cyclopentylen, 1,2-, 1,3- oder 1,4-Phenylen, 4,4'-Biphenylen, 1,4-Diaza-1,4-butylen, 1-Aza-1,3-propylen, 1,4,7-Triaza-1,7-heptylen, 1,4-Diaza-1,6-hexylen, 1,4-Diaza-7-oxa-1,7-heptylen, 4,7-Diaza-1-oxa-1,7-heptylen, 4-Aza-1-oxa-1,6-hexylen, 1-Aza-4-oxa-1,4-butylen, 1-Aza-1,3-propylen, 4-Aza-1-oxa-1,4-butylen, 4-Aza-1,7-dioxa-1,7-heptylen, 4-Aza-1-oxa-4-methyl-1,6-hexylen, 4-Aza-1,7-dioxa-4-methyl-1,7-heptylen, 4-Aza-1,7-dioxa-4-(2'-hydroxyethyl)-1,7-heptylen, 4-Aza-1-oxa-(2'-hydroxyethyl)-1,6-hexylen oder 1,4-Piperazinylen und
gegebenenfalls durch Aryl, Alkyl, Aryloxy, Alkyloxy, Heteroatome und/oder Heterocyclen substituiertes C₆-C₁₂-Arylen beispielsweise 1,2-, 1,3- oder 1,4-Phenylen, 4,4'-Biphenylen, Toluylen oder Xylylen.

Beispiele für R¹ sind 1,2-Ethylen, 1,2-Propylen, 1,1-Dimethyl-1,2-ethylen, 1-Hydroxymethyl-1,2-ethylen, 2-Hydroxy-1,3-propylen, 1,3-Propylen, 1,4-Butylen, 1,5-Pentylen, 2-Methyl-1,3-propylen, 2-Ethyl-1,3-propylen, 2,2-Dimethyl-1,3-propylen und 2,2-Dimethyl-1,4-butylen, bevorzugt sind 1,4-Butylen, 1,5-Pentylen und 1,3-Propylen, besonders bevorzugt ist 1,3-Propylen.

Beispiele für R² sind 1,2-Ethylen, 1,2-Propylen, 1,1-Dimethyl-1,2-ethylen, 1-Hydroxymethyl-1,2-ethylen, 2-Hydroxy-1,3-propylen, 1,3-Propylen, 1,4-Butylen, 1,6-Hexylen, 2-Methyl-1,3-propylen, 2-Ethyl-1,3-propylen, 2,2-Dimethyl-1,3-propylen und 2,2-Dimethyl-1,4-butylen, 1,2-Cyclopentylen, 1,3-Cyclopentylen, 1,2-Cyclohexylen,1,3-Cyclohexylen, ortho-Phenylen, 3-Oxa-1,5-pentylen, 3,6-Dioxa-1,8-octylen und 3,6,8-Trioxa-1,8,11-undecylen, bevorzugt sind 1,2-Ethylen, 1,2-Propylen, 1,3-Propylen, besonders bevorzugt sind 1,2-Ethylen und 1,2-Propylen und ganz besonders bevorzugt ist 1,2-Ethylen.

Bevorzugte Individuen (L) sind N-(2-Hydroxyethyl)-pyrrolidon, N-(2-Hydroxypropyl)-pyrrolidon, N-(2'-(2-Hydroxyethoxy)-ethyl)-pyrrolidon, N-(2-Hydroxyethyl)-caprolactam, N-(2-Hydroxypropyl)-caprolactam sowie N-(2'-(2-Hydroxyethoxy)-ethyl)-caprolactam, bevorzugt sind N-(2-Hydroxyethyl)-pyrrolidon und N-(2-Hydroxypropyl)-pyrrolidon, besonders bevorzugt ist N-(2-Hydroxyethyl)-pyrrolidon.

Soweit die N-hydroxyalkylierten Lactame (L) optisch aktiv sind, werden sie bevorzugt racemisch oder als Diastereomerengemisch eingesetzt, es ist jedoch auch möglich, sie als reine Enantiomere bzw. Diastereomere oder als Enantiomerengemische einzusetzen.

Im Reaktionsschritt erfolgt die Veresterung mit (Meth)acrylsäure (S) oder bevorzugt die Umesterung mit mindestens einem, bevorzugt genau einem (Meth)acrylsäureester (D) erfindungsgemäß in Anwesenheit mindestens eines Katalysators (K) ausgewählt aus der Gruppe bestehend aus Alkalimetallhydroxiden, Erdalkalimetallhydroxiden und Metallacetylacetonaten.

Zur Veresterung können (Meth)acrylsäure (S) oder zur Umesterung (Meth)acrylsäureester (D) eines gesättigten Alkohols eingesetzt werden, bevorzugt gesättigte C₁-C₁₀-Alkylester oder C₃-C₁₂-Cycloalkylester der (Meth)acrylsäure, besonders bevorzugt gesättigte C₁-C₄-Alkylester der (Meth)acrylsäure.

Gesättigt bedeutet im Rahmen dieser Schrift Verbindungen ohne C-C-Mehrfachbindungen (außer selbstverständlich die C=C-Doppelbindung in den (Meth)acryleinheiten).

Beispiele für Verbindungen (D) sind (Meth)acrylsäuremethyl-, -ethyl-, -n-butyl-, -isobutyl-, -tert-butyl-, n-octyl- und -2-Ethylhexylester, 1,2-Ethylenglycoldi- und -mono(meth)acrylat, 1,4-Butandioldi- und -mono(meth)acrylat, 1,6-Hexandioldi- und -mono(meth)acrylat, Trimethylolpropantri(meth)acrylat und Pentaerythrittetra(meth)acrylat.

Besonders bevorzugt sind (Meth)acrylsäuremethyl-, -ethyl-, -n-butyl- und -2-Ethylhexylester, ganz besonders bevorzugt (Meth)acrylsäuremethyl-, -ethyl- und -n-butylester, insbesondere (Meth)acrylsäuremethyl- und -ethylester und speziell (Meth)acrylsäuremethylester.

Erfindungsgemäß einsetzbare Katalysators (K) sind ausgewählt aus der Gruppe bestehend aus Alkalimetallhydroxiden, Erdalkalimetallhydroxiden und Metallacetylacetonaten.

Diese Katalysatoren können sowohl homogen als auch heterogen sein. Heterogene Katalysatoren sind im Rahmen dieser Schrift erfindungsgemäß solche, die eine Löslichkeit im Reaktionsmedium bei 25 °C von nicht mehr als 1 g/l aufweisen, bevorzugt von nicht mehr als 0,5 g/l und besonders bevorzugt von nicht mehr als 0,25 g/l.

Unter Alkalimetall- und Erdalkalimetallhydroxiden im Sinne der vorliegenden Anmeldung werden basische Verbindungen verstanden, die einen pK_{B}-Wert von nicht mehr als 7,0, bevorzugt nicht mehr als 6,0 und besonders bevorzugt nicht mehr als 4,0 aufweisen.

Die Alkalimetall- und Erdalkalimetallhyxdroxide können sowohl in fester Form als auch in Form von Lösungen, beispielsweise als wässrige Lösungen, verwendet werden. Bevorzugt werden die Alkalimetall- und Erdalkalimetallhydroxide in fester Form dem erfindungsgemäßen Verfahren zugesetzt.

Geeignete Alkalimetallhydroxide sind beispielsweise Lithiumhydroxid, Natriumhydroxid und Kaliumhydroxid. Geeignete Erdalkalimetallhydroxide sind beispielsweise Magnesiumhydroxid und Calciumhydroxid. Bevorzugt sind Alkalimetallhydroxide wie Lithiumhydroxid, Natriumhydroxid und Kaliumhydroxid.

Ebenfalls geeignete Katalysatoren (K) sind Metallacetylacetonate. Metallacetylacetonate sind Metall-Chelate mit dem Enolat-Anion von 2,4-Pentandion (Acetylaceton) und haben die allgemeine Formel Mₙ(C₅H₇O₂)ₙ, bzw. Mₙ(acac)ₙ. Als Metalle M kommen zahlreiche Metalle, insbesondere Übergangsmetalle in Frage. Bevorzugt sind Metalle M ausgwählt aus der Gruppe der Übergangsmetalle, Alkalimetalle, Erdalkalimetalle und Aluminium. Geeignete Alkali- und Erdalkalimetalle sind beispielsweise Lithium, Natrium, Kalium, Magnesium und Calcium. Bevorzugt werden Aluminium, Lithium, Natrium, Kalium und Calcium eingesetzt. Als Übergangsmetalle werden bevorzugt Titan, Zirconium, Chrom, Mangan, Cobalt, Nickel und Kupfer verwendet. Bevorzugte Vertreter dieser Gruppe sind Lithiumacetylacetonat (Li(acac)), Natriumacetylacetonat (Na(acac)) und Kaliumacetylacetonat (K(acac)) sowie Calciumacetylacetonat (Ca(acac)₂).

Die durch den Katalysator (K) katalysierte Ver- oder Umesterung erfolgt im Allgemeinen bei 30 bis 140 °C, bevorzugt bei 30 bis 100 °C, besonders bevorzugt bei 40 bis 90 °C und ganz besonders bevorzugt bei 50 bis 80 °C.

In einer bevorzugten Ausführungsform des erfindungsgemäßen Verfahrens wird die Reaktion unter leichtem Vakuum von beispielsweise 200 hPa bis Normaldruck, bevorzugt 200 bis 600 hPa und besonders bevorzugt 300 bis 500 hPa durchgeführt werden, wenn das bei der Veresterung freigesetzte Wasser oder der bei der Umesterung entstehende niedrigsiedende Alkohol, gegebenenfalls als Azeotrop, abdestilliert werden soll.

Das molare Verhältnis zwischen (Meth)acrylsäure (S) oder (Meth)acrylsäureester (D) und N-hydroxyalkyliertem Lactam (L) beträgt bei der durch einen Katalysator (K) katalysierten Ver- oder Umesterung in der Regel 1 - 6 : 1 mol/mol, bevorzugt 1 - 5 : 1 mol/mol und besonders bevorzugt 1 - 4 : 1 mol/mol.

Die Reaktionszeit bei der durch einen Katalysator (K) katalysierten Ver- oder Umesterung beträgt in der Regel 45 min bis 18 Stunden, bevorzugt 2 Stunden bis 12 Stunden und besonders bevorzugt 3 bis 10 Stunden.

Der Gehalt an Katalysator (K), der ausgewählt ist aus der Gruppe der Alkalimetallhydroxide, Erdalkalimetallhydroxide und Metallacetylacetonate, im Reaktionsmedium liegt in der Regel im Bereich von etwa 0,01 bis 5 mol%, bevorzugt 0,1 - 3 und besonders bevorzugt 0,3 - 2 mol% bezogen auf die Summe der eingesetzten N-hydroxyalkylierten Lactame (L).

Bei der Ver- oder Umesterung sind Polymerisationsinhibitoren (wie unten beschrieben) zwingend erforderlich.

Die Anwesenheit von sauerstoffhaltigen Gasen (s.u.) während der durch einen Katalysator (K) katalysierten Reaktion ist bevorzugt.

In einer bevorzugten Ausführungsform des erfindungsgemäßen Verfahrens wird deshalb während der Reaktion kontinuierlich ein sauerstoffhaltiges Gas in das Reaktionsgemisch eingeleitet. Im Falle der weiteren Aufarbeitung des Rohprodukts, beispielsweise durch Destillation oder Rektifikation, ist ebenfalls die kontinuierliche Sauerstoffeinleitung bevorzugt.

Bei der erfindungsgemäßen Ver- oder Umesterung werden in der Regel die Produkte mit einer Farbzahl unter 500 APHA, bevorzugt unter 200 und besonders bevorzugt unter 150 (gemäß DIN ISO 6271) erhalten.

Die Reaktion kann in organischen Lösungsmitteln oder deren Gemischen oder ohne Zusatz von Lösungsmitteln ablaufen. Die Ansätze sind in der Regel weitgehend wasserfrei, d. h. der Wassergehalt beträgt bevorzugt unter 10, besonders bevorzugt unter 5, insbesondere bevorzugt unter 1 und ganz besonders bevorzugt unter 0,5 Gew.-%. Im Regelfall beträgt der Wassergehalt zwischen 100 und 5000 ppm, bevorzugt zwischen 500 und 1000 ppm. Ferner sind die Ansätze weitgehend frei von primären und sekundären Alkoholen, d.h. unter 10, bevorzugt unter 5, besonders bevorzugt unter 1 und ganz besonders bevorzugt unter 0,5 Gew.-% Alkoholgehalt.

Geeignete organische Lösungsmittel sind solche für diese Zwecke bekannten, beispielsweise tertiäre Monoole, wie C₃-C₆-Alkohole, bevorzugt tert-Butanol, tert-Amylalkohol, Pyridin, Poly-C₁-C₄-alkylenglykoldi-C₁-C₄-alkylether, bevorzugt Polyethylenglycoldi-C₁-C₄-alkylether, wie z.B. 1,2-Dimethoxyethan, Diethylenglycoldimethylether, Polyethylenglycoldimethylether 500, C₁-C₄-Alkylencarbonate, insbesondere Propylencarbonat, C₃-C₆-Alkylessigsäureester, insbesondere tert.-Butylessigsäureester, THF, Toluol, 1,3-Dioxolan, Aceton, iso-Butyl-methylketon, Ethylmethylketon, 1,4-Dioxan, tert-Butylmethylether, Cyclohexan, Methylcyclohexan, Toluol, Hexan, Dimethoxymethan, 1,1-Dimethoxyethan, Acetonitril, sowie deren ein- oder mehrphasige Mischungen.

In einer besonders bevorzugten Ausführungsform der Umesterung wird die Reaktion in dem als Edukt eingesetzten (Meth)acrylsäureester (D) durchgeführt. Ganz besonders bevorzugt ist die Durchführung der Reaktion in der Weise, dass das Produkt (F) nach Beendigung der Reaktion als etwa 10 - 80 Gew.-%ige Lösung in dem als Edukt eingesetzten (Meth)acrylsäureester (D) anfällt, insbesondere als 20 bis 50 Gew.-%ige Lösung.

Die Edukte liegen entweder gelöst, als Feststoffe suspendiert oder in Emulsion im Reaktionsmedium vor. In einer bevorzugten Ausführungsform des erfindungsgemäßen Verfahrens wird der Katalysator (K), der ausgewählt ist aus der Gruppe der Alkalimetallhydroxide, Erdalkalimetallhydroxide und Metallacetylacetonate, in Abwesenheit von Lösungsmitteln und bevorzugt als Feststoff eingesetzt.

Die Reaktion kann kontinuierlich, beispielsweise in einem Rohrreaktor oder in einer Rührreaktorkaskade, oder diskontinuierlich erfolgen. In einer bevorzugten Ausführungsform des erfindungsgemäßen Verfahrens werden jedoch alle Edukte, sowie Stabilisatoren und der Katalysator (K) vollständig zu Beginn der Reaktion zugesetzt, d.h. nicht kontinuierlich während des Reaktionsverlaufs.

Die Umsetzung kann in allen für eine solche Umsetzung geeigneten Reaktoren durchgeführt werden. Solche Reaktoren sind dem Fachmann bekannt. Bevorzugt erfolgt die Umsetzung in einem Rührkesselreaktor oder einem Festbettreaktor.

Zur Durchmischung des Reaktionsansatzes können beliebige Verfahren eingesetzt werden. Spezielle Rührvorrichtungen sind nicht erforderlich. Die Durchmischung kann beispielsweise durch Einspeisen eines Gases, bevorzugt eines sauerstoffhaltigen Gases (siehe unten) erfolgen. Das Reaktionsmedium kann ein- oder mehrphasig sein und die Reaktanden werden darin gelöst, suspendiert oder emulgiert. Die Temperatur wird während der Reaktion auf den gewünschten Wert eingestellt und kann, falls gewünscht, während des Reaktionsverlaufs erhöht oder verringert werden.

Die Entfernung von Wasser im Falle einer Veresterung oder Alkoholen, die bei einer Umesterung aus den (Meth)acrylsäureestern (D) freigesetzt werden, erfolgt kontinuierlich oder schrittweise in an sich bekannter Weise, z.B durch Vakuum, azeotrope Entfernung, Strippen, Absorption, Pervaporation und Diffusion über Membranen oder Extraktion.

Vorteilhaft wird die Ver- oder Umesterung in Gegenwart eines sauerstoffhaltigen Gases, bevorzugt Luft oder Luft-Stickstoff-Gemische, durchgeführt.

Dieses Strippen kann beispielsweise durch Durchleiten eines sauerstoffhaltigen Gases, bevorzugt eines Luft oder Luft-Stickstoff-Gemisches, durch das Reaktionsgemisch erfolgen, gegebenenfalls zusätzlich zu einer Destillation. Wie bereits oben beschrieben wird in einer bevorzugten Ausführungsform des erfindungsgemäßen Verfahrens während der Reaktion kontinuierlich ein sauerstoffhaltiges Gas in das Reaktionsgemisch eingeleitet.
Zur Absorption eignen sich vorzugsweise Molekularsiebe oder Zeolithe (Porengröße z. B. im Bereich von etwa 3-10 Angström), eine Abtrennung durch Destillation oder mit Hilfe geeigneter semipermeabler Membranen.

Es ist aber auch möglich, das abgetrennte Gemisch aus (Meth)acrylsäureester (D) und dem diesem zugrundeliegenden Alkohol, das häufig ein Azeotrop bildet, direkt in eine Anlage zur Herstellung des (Meth)acrylsäureesters (D) zuzuführen, um es dort in einer Veresterung mit (Meth)acrylsäure wiederzuverwerten.

Nach Beendigung der Reaktion kann man das aus der Ver- oder Umesterung erhaltene Reaktionsgemisch ohne weitere Aufreinigung verwenden oder es erforderlichenfalls in einem weiteren Schritt aufreinigen.

In der Regel wird im Aufreinigungsschritt lediglich der eingesetzte Katalysator (K) vom Reaktionsgemisch abgetrennt und das Reaktionsprodukt vom gegebenenfalls verwendeten organischen Lösungsmittel abgetrennt.

Eine Abtrennung vom Katalysator (K) erfolgt in der Regel durch Filtration, Elektrofiltration, Absorption, Zentrifugation oder Dekantieren oder durch Destillation oder Rektifikation. Der abgetrennte Katalysator (K) kann anschließend für weitere Reaktionen eingesetzt werden.

Die Abtrennung vom organischen Lösungsmittel erfolgt in der Regel durch Destillation, Rektifikation oder bei festen Reaktionsprodukten durch Filtration.

Bevorzugt werden im Aufreinigungsschritt jedoch lediglich der Katalysator (K) und das gegebenenfalls eingesetzte Lösungsmittel abgetrennt.

Das gegebenenfalls aufgereinigte Reaktionsgemisch wird bevorzugt einer Destillation unterworfen, in der der (Meth)acrylsäureester (F) der N-hydroxyalkylierten Lactame destillativ von unumgesetzer (Meth)acrylsäure (S) oder unumgesetztem (Meth)acrylsäureester (D) sowie gegebenenfalls gebildeten Nebenprodukten getrennt wird. Wie bereits oben beschrieben wird bei einer Aufarbeitung des Rohprodukts durch Destillation oder Rektifikation ebenfalls die kontinuierliche Sauerstoffeinleitung bevorzugt.

Bei den Destillationseinheiten handelt es sich zumeist um Rektifikationskolonnen üblicher Bauart mit Umlaufverdampfer und Kondensator. Der Zulauf erfolgt in bevorzugt den Sumpfbereich, die Sumpftemperatur beträgt hier beispielsweise 130 - 160 °C, bevorzugt 150 - 160 °C, die Kopftemperatur bevorzugt 140 - 145 °C und der Kopfdruck 3 - 20, bevorzugt 3 bis 5 mbar. Selbstverständlich kann der Fachmann auch andere Temperatur- und Druckbereiche ermitteln, in denen die jeweiligen (Meth)acrylsäureester (F) der N-hydroxyalkylierten Lactame destillativ gereinigt werden können. Wesentlich ist dabei eine Trennung des Wunschproduktes von Edukten und Nebenprodukten unter Bedingungen, bei denen das Wunschprodukt möglichst keiner Abbaureaktion ausgesetzt ist.

Die Destillationseinheit weist in der Regel 5 bis 50 theoretische Böden auf.

Die Destillationseinheiten sind von an sich bekannter Bauart und weisen die üblichen Einbauten auf. Als Kolonneneinbauten kommen prinzipiell alle gängigen Einbauten in Betracht, beispielsweise Böden, Packungen und/oder Schüttungen. Von den Böden sind Glockenböden, Siebböden, Ventilböden, Thormannböden und/oder Dual-Flow-Böden bevorzugt, von den Schüttungen sind solche mit Ringen, Wendeln, Sattelkörpern, Raschig-, Intos- oder Pall-Ringen, Barrel- oder Intalox-Sätteln, Top-Pak etc. oder Geflechten bevorzugt.

Bevorzugt wird das Wunschprodukt diskontinuierlich destilliert, wobei zunächst Leichtsieder aus dem Reaktionsgemisch entfernt werden, zumeist Lösungsmittel oder unumgesetzte (Meth)acrylsäure (S) oder (Meth)acrylsäureester (D). Nach Abtrennung dieser Leichtsieder wird die Destillationstemperatur erhöht und/oder das Vakuum verringert und das Wunschprodukt abdestilliert.

Der verbleibende Destillationsrückstand wird zumeist verworfen.

Die Reaktionsbedingungen bei der erfindungsgemäßen Ver- oder Umesterung sind mild. Aufgrund der niedrigen Temperaturen und sonstigen milden Bedingungen wird die Bildung von Nebenprodukten in der Reaktion vermieden, die andernfalls zum Beispiel von stark sauren oder basischen Katalysatoren stammen können oder durch unerwünschte radikalische Polymerisation der eingesetzten (Meth)acrylverbindung (B) entstehen können, die sonst nur durch Zugabe von Stabilisatoren verhindert werden kann.

Bei der erfindungsgemäßen Reaktionsführung kann der Reaktionsmischung über den ohnehin in der (Meth)acrylverbindung (B) enthaltenen Lagerstabilisator hinaus zusätzlicher Stabilisator zugegeben werden, beispielsweise Hydrochinonmonomethylether, Phenothiazin, Phenole, wie z.B. 2-tert.-Butyl-4-methylphenol, 6-tert.-Butyl-2,4-dimethyl-phenol oder N-Oxyle, wie 4-Hydroxy-2,2,6,6-tetramethyl-piperidin-N-oxyl, 4-Oxo-2,2,6,6-tetramethyl-piperidin-N-oxyl oder Uvinul^{®} 4040P der BASF Aktiengesellschaft oder Amine wie Kerobit^{®} BPD der BASF Aktiengesellschaft (N,N'-di-sec.-butyl-p-phenylendiamin), beispielsweise in Mengen von 50 bis 2000 ppm.

Die erfindungsgemäß eingesetzten Katalysatoren (K) zeigen eine lediglich geringe Tendenz zu Nebenreaktionen.

Des weiteren ist die Reaktion unter den erfindungsgemäßen Reaktionsbedingungen sehr selektiv, man findet in der Regel weniger als 10 %, bevorzugt weniger als 5 % Nebenprodukte (bezogen auf den Umsatz).

Die erfindungsgemäß hergestellten (Meth)acrylsäureester (F) von N-hydroxyalkylierten Lactamen finden Anwendung beispielsweise als Monomere oder Comonomere in der Herstellung von Dispersionen, beispielsweise Acryldispersionen, als Reaktivverdünner, beispielsweise in strahlungshärtbaren Beschichtungsmassen oder in Farben, bevorzugt in Außenfarben, sowie in Dispersionen für Anwendung im Papierbereich.

Die folgenden Beispiele sollen die Eigenschaften der Erfindung erläutern, ohne sie aber einzuschränken.

### Beispiele

Als "Teile" seien in dieser Schrift, wenn nichts anderes angegeben, "Gewichtsteile" verstanden.

### Beispiel 1

### Herstellung von N-(2-Hydroxyethyl)-pyrroldion-methacrylsäureester mit Lithiumhydroxid als Katalysator mit anschließender Reindestillation

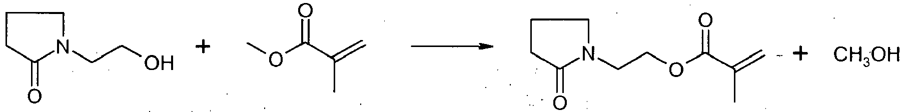

Die Umesterung erfolgte in einem 750 mL Miniplantreaktor mit Oldershaw-Kolonne und Flüssigkeitsverteiler. Das Rücklaufverhältnis betrug 25 : 1 (Rücklauf : Ablauf), die Rührgeschwindigkeit (Anker-Rührer) 400 U/min und die Lufteinleitung 1,5 L/h.

In dieser Apparatur wurden 280 mg Hydrochinonmonomethylether(350 ppm), 40 mg Phenothiazin (50 ppm), 600 g (6,0 mol) Methylmethacrylat (MMA) und 194 g (1,5 mol) Hydroxyethylpyrrolidon vorgelegt und gerührt. Anschließend wurden 0,72 g (30 mmol; 2,0 mol-% bezogen auf Hydroxyethylpyrrolidon) festes Lithiumhydroxid zugegeben, das Vakuum eingestellt (300 mbar) und die Suspension erhitzt (die Manteltemperatur wurde mittels Thermostaten auf 120 °C eingestellt). Nach ca. 10 Minuten begann die Suspension zu sieden, dieser Zeitpunkt wurde als Startpunkt gewählt (t = 0 min). Während der Reaktion wurde kontinuierlich Destillat (Azeotrop aus MMA und Methanol) entfernt. Nach 300 min wurde die Reaktion beendet und das Vakuum aufgehoben. Die Suspension wurde abgekühlt und anschließend über eine Druckfilternutsche filtriert. Man erhielt 494 g Rohprodukt.
Anschließend wurden 247 g des Rohprodukts 200 ppm Kerobit^{®} BPD (BASF Aktiengesellschaft, N,N'-di-sec.-butyl-p-phenylendiamin) zur Stabilisierung zugegeben. Die Mischung wurde unter Vakuum destilliert, wobei zunächst überschüssiges Methylmethacrylat entfernt wurde, und anschließend wurde das Wertprodukt erhalten (131-132 °C
bei 1,3 mbar). Während der Destillation wurde Sauerstoff in das zu destillierende Gemisch eingeleitet.

Man erhielt 133,5 g (90 % Ausbeute) Produkt mit folgender Zusammensetzung (GC-Analyse): 97,8 % N-(2-Hydroxyethyl)-pyrrolidon-methacrylsäureester, 0,6 % Hydroxyethylpyrrolidon, 0,2 % Methylmethacrylat und 1,4 % sonstige Nebenprodukte. Die APHA-Farbzahl betrugt 67. Im Sumpf verblieben 6 g Rückstand.

### Beispiel 2

### Herstellung von N-(2-Hydroxyethyl)-pyrroldion-methacrylsäureester mit Natriumhydroxid als Katalysator mit anschließender Reindestillation

Beispiel 1 wurde analog nachgearbeitet, jedoch wurden anstatt Lithiumhydroxyid 1,2 g (30 mmol; 2,0 mol-% bezogen auf Hydroxyethylimidazol) festes Natriumhydroxid als Katalysator eingesetzt. Die Reaktion wurde nach 120 min beendet und das Vakuum aufgehoben. Dis Suspension wurde abgekühlt und anschließend über eine Druckfilternutsche filitriert. Man erhielt 644 g Rohprodukt.

Anschließend wurden dem Rohprodukt 200 ppm Kerobit^{®} BPD (BASF Aktiengesellschaft, N,N'-di-sec.-butyl-p-phenylendiamin) zur Stabilisierung zugegeben. Die Mischung wurde unter Vakuum destilliert, wobei zunächst überschüssiges Methylmethacrylat entfernt wurde, und anschließend wurde das Wertprodukt erhalten (142-144 °C bei 2,8 mbar). Während der Destillation wurde Sauerstoff in das zu destillierende Gemisch eingeleitet.

Man erhielt 258 g (87 % Ausbeute) Produkt mit folgender Zusammensetzung (GC-Analyse): 95,5 % N-(2-Hydroxyethyl)-pyrrolidon-methacrylsäureester, 0,6 % Hydroxyethylpyrrolidon, 0,1 % Methylmethacrylat und 1,4 % sonstige Nebenprodukte. Die APHA-Farbzahl betrugt 50. Im Sumpf verblieben 14 g Rückstand.

### Beispiel 3

### Herstellung von N-(2-Hydroxyethyl)-pyrroldion-methacrylsäureester mit Kaliumhydroxid als Katalysator mit anschließender Reindestillation

Beispiel 1 wurde analog nachgearbeitet, jedoch wurden anstatt Lithiumhydroxyid 1,68 g (30 mmol; 2,0 mol-% bezogen auf Hydroxyethylimidazol) festes Kaliumhydroxid als Katalysator eingesetzt. Die Reaktion wurde nach 120 min beendet und das Vakuum aufgehoben. Dis Suspension wurde abgekühlt und anschließend über eine Druckfilternutsche filitriert. Man erhielt 636 g Rohprodukt.

Anschließend wurden dem Rohprodukt 200 ppm Kerobit^{®} BPD (BASF Aktiengesellschaft, N,N'-di-sec.-butyl-p-phenylendiamin) zur Stabilisierung zugegeben. Die Mischung wurde unter Vakuum destilliert, wobei zunächst überschüssiges Methylmethacrylat entfernt wurde, und anschließend wurde das Wertprodukt erhalten (156-158 °C bei 5,5 mbar). Während der Destillation wurde Sauerstoff in das zu destillierende Gemisch eingeleitet.

Man erhielt 258 g (79 % Ausbeute) Produkt mit folgender Zusammensetzung (GC-Analyse): 98,5 % N-(2-Hydroxyethyl)-pyrrolidon-methacrylsäureester, 0,5 % Hydroxyethylpyrrolidon und 1,4 % sonstige Nebenprodukte. Methalmethacrylat war nicht mehr enthalten. Die APHA-Farbzahl betrugt 58. Im Sumpf verblieben 45 g Rückstand.

### Beispiel 4

### Herstellung von N-(2-Hydroxyethyl)-pyrroldion-methacrylsäureester mit Lithiumhydroxid als Katalysator ohne Phenothiazin als Stabilisator und ohne anschließende Reindestillation

In der in Beispiel 1 beschriebenen Apparatur wurden 280 mg Hydrochinonmonomethylether(350 ppm), 600 g (6,0 mol) Methylmethacrylat (MMA) und 194 g (1,5 mol) Hydroxyethylpyrrolidon vorgelegt und gerührt. Anschließend wurden 1,08 g (45 mmol; 3,0 mol-% bezogen auf Hydroxyethylpyrrolidon) festes Lithiumhydroxid zugegeben, das Vakuum eingestellt (300 mbar) und die Suspension erhitzt (die Manteltemperatur wurde mittels Thermostaten auf 120 °C eingestellt). Nach ca. 10 Minuten begann die Suspension zu sieden, dieser Zeitpunkt wurde als Startpunkt gewählt (t = 0 min). Während der Reaktion wurde kontinuierlich Destillat (Azeotrop aus MMA und Methanol) entfernt. Nach 180 min wurde die Temperatur im Sumpf auf 60 °C eingestellt und überschüssiges MMA im Vakuum entfernt. Anschließend wurde das Vakuum aufgehoben, die Suspension abgekühlt und anschließend über eine Druckfilternutsche filtriert. Man erhielt 287 g Rohprodukt (Ausbeute 97 %) als leicht gelbliche Flüssigkeit mit folgender Zusammensetzung (GC-Analyse): 96,5 % N-(2-Hydroxyethyl)-pyrrolidonmethacrylsäureester, 0,4 % Hydroxyethylpyrrolidon, 0,7 % Methylmethacrylat und 2,3 % sonstige Nebenprodukte. Die APHA-Farbzahl betrugt 125.

### Beispiel 5

### Herstellung von N-(2-Hydroxyethyl)-pyrroldion-methacrylsäureester mit Kaliumhydroxid als Katalysator ohne Hydrochinonmonomethylether und Phenothiazin als Stabilisatoren und ohne anschließende Reindestillation

In der in Beispiel 1 beschriebenen Apparatur wurden 308 g (3,1 mol) Methylmethacrylat (MMA), das mit 15 ppm Hydrochinonmonomethylether stabilisiert ist, und 100 g (0,77 mol) Hydroxyethylpyrrolidon (APHA-Farbzahl 118) vorgelegt und gerührt. Anschließend wurden 0,86 g (15 mmol; 2,0 mol-% bezogen auf Hydroxyethylpyrrolidon) festes Kaliumhydroxid zugegeben, das Vakuum eingestellt (300 mbar) und die Suspension erhitzt (die Manteltemperatur wurde mittels Thermostaten auf 120 °C eingestellt). Nach ca. 10 Minuten begann die Suspension zu sieden, dieser Zeitpunkt wurde als Startpunkt gewählt (t = 0 min). Während der Reaktion wurde kontinuierlich Destillat (Azeotrop aus MMA und Methanol) entfernt. Nach 150 min wurde die Temperatur im Sumpf auf 60 °C eingestellt und überschüssiges MMA im Vakuum entfernt. Anschließend wurde das Vakuum aufgehoben, die Suspension abgekühlt und anschließend über eine Druckfilternutsche filtriert. Man erhielt 143 g Rohprodukt (Ausbeute 94 %) als leicht gelbliche Flüssigkeit mit folgender Zusammensetzung (GC-Analyse): 95,3 % N-(2-Hydroxyethyl)-pyrrolidon-methacrylsäureester, 1,1 % Hydroxyethylpyrrolidon, 1,7 % Methylmethacrylat und 2,0 % sonstige Nebenprodukte. Die APHA-Farbzahl betrugt 92.

### Beispiel 6

### Herstellung von N-(2-Hydroxyethyl)-pyrroldion-methacrylsäureester mit Natriumacetylacetonat als Katalysator mit anschließender Reindestillation

Beispiel 1 wurde analog nachgearbeitet, jedoch wurden anstatt Lithiumhydroxyid 4,2 g (30 mmol; 2,0 mol-% bezogen auf Hydroxyethylimidazol) festes Natriumacetylacetonat als Katalysator eingesetzt. Die Reaktion wurde nach 180 min beendet und das Vakuum aufgehoben. Dis Suspension wurde abgekühlt und anschließend über eine Druckfilternutsche filitriert. Man erhielt 457 g Rohprodukt.

Anschließend wurden 448 g des Rohprodukts 200 ppm Kerobit^{®} BPD (BASF Aktiengesellschaft, N,N'-di-sec.-butyl-p-phenylendiamin) zur Stabilisierung zugegeben. Die Mischung wurde unter Vakuum destilliert, wobei zunächst überschüssiges Methylmethacrylat entfernt wurde, und anschließend wurde das Wertprodukt in zwei Fraktionen erhalten (148-149 °C bei 4 mbar). Während der Destillation wurde Sauerstoff in das zu destillierende Gemisch eingeleitet. Im Sumpf verblieben 39 g Rückstand.

Man erhielt 229 g (79 % Ausbeute) Produkt mit folgender Zusammensetzung (GC-Analyse) und Farbzahl:

| Fraktion | Produkt¹⁾ [%] | HEP²⁾ [%] | MMA [%] | Nebenprodukte³⁾ [%] | Farbzahl [APHA] |
|---|---|---|---|---|---|
| 1 (183 g) | 95,1 | 2,7 | 0,5 | 1,7 | 63 |
| 2 (46 g) | 92,8 | 3,5 | 0,5 | 3,2 | 79 |

| | | | | | |
|---|---|---|---|---|---|
| ¹⁾ N-(2-Hydroxyethyl)-pyrroldion-methacrylsäureester ²⁾ Hydroxyethylpyrrolidon ³⁾ Summe der sonstigen Nebenprodukte | | | | | |

## Patentansprüche

1. Verfahren zur Herstellung von (Meth)acrylsäureestern (F) von N-hydroxyalkylierten Lactamen, in dem man ringförmige N-hydroxyalkylierte Lactame (L), 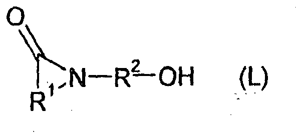 worin
R¹ C₁-C₅-Alkylen oder durch ein- oder mehrere Sauerstoff- und/oder Schwefelatome und/oder ein oder mehrere substituierte oder unsubstituierte Iminogruppen und/oder durch eine öder mehrere Cycloalkyl-, -(CO)-, -O(CO)O-, -(NH)(CO)O-, -O(CO)(NH)-, -O(CO)- oder -(CO)O-Gruppen unterbrochenes C₂-C₂₀-Alkylen, wobei die genannten Reste jeweils durch Aryl, Alkyl, Aryloxy, Alkyloxy, Heteroatome und/oder Heterocyclen substituiert sein können
mit der Maßgabe, dass R¹ kein anderes Atom als ein Kohlenstoffatom direkt benachbart zur Lactam-Carbonylgruppe aufweisen darf,
R² C₁-C₂₀-Alkylen, C₅-C₁₂-Cycloalkylen, C₆-C₁₂-Arylen oder durch ein- oder mehrrere Sauerstoff- und/oder Schwefelatome und/oder ein oder mehrere substituierte oder unsubstituierte Iminogruppen und/oder durch eine oder mehrere Cycloalkyl-, -(CO)-, -O(CO)O-, -(NH)(CO)O-, -O(CO)(NH)-, -O(CO)- oder -(CO)O-Gruppen unterbrochenes C₂-C₂₀-Alkylen, wobei die genannten Reste jeweils durch Aryl, Alkyl, Aryloxy, Alkyloxy, Heteroatome und/oder Heterocyclen substituiert sein können, oder
R²-OH für eine Gruppe der Formel -[Xᵢ]ₖ-H,
k für eine Zahl von 1 bis 50 und
Xᵢ für jedes i = 1 bis k unabhängig voneinander ausgewählt sein kann aus der Gruppe -CH₂-CH₂-O-, -CH₂-CH(CH₃)-O-, -CH(CH₃)-CH₂-O-, -CH₂-C(CH₃)₂-O-, -C(CH₃)₂-CH₂-O-, -CH₂-CH₂-CH₂-O-, -CH₂-CH₂-CH₂-CH₂-O-, -CH₂-CHVin-O-, -CHVin-CH₂-O-, -CH₂-CHPh-O- und -CHPh-CH₂-O-, worin Ph für Phenyl und Vin für Vinyl steht,
bedeuten,
in Gegenwart mindestens eines Katalysators (K) ausgewählt aus der Gruppe bestehend aus Alkalimetallhydroxiden, Erdalkalimetallhydroxiden und Metallacetylacetonaten und eines Polymerisationsinhibitors mit (Meth)acrylsäure (S) verestert oder mit mindestens einem (Meth)acrylsäureester (D) umestert.

2. Verfahren nach Anspruch 1, **dadurch gekennzeichnet, dass** die Alkalimetallhydroxide ausgewählt sind aus der Gruppe bestehend aus Lithiumhydroxid, Natriumhydroxid und Kaliumhydroxid, und die Erdalkalimetallhydroxide ausgewählt sind aus der Gruppe bestehend aus Magnesiumhydroxid und Calciumhydroxid

3. Verfahren nach einem der Ansprüche 1 oder 2, **dadurch gekennzeichnet, dass** als Katalysator (K) Alkalimetallhydroxide ausgewählt aus der Gruppe bestehend aus Lithiumhydroxid, Natriumhydroxid und Kaliumhydroxid eingesetzt werden.

4. Verfahren nach Anspruch 1, **dadurch gekennzeichnet, dass** als Katalysatoren (K) Metallacetylacetonate der allgemeine Formel Mₙ(C₅H₇O₂)ₙ bzw. Mₙ(acac)ₙ eingesetzt werden, worin M ausgewählt ist aus der Gruppe der Übergangsmetalle, Alkalimetalle, Erdalkalimetalle und Aluminium.

5. Verfahren nach Anspruch 1 oder 4, **dadurch gekennzeichnet, dass** als Metallacetylacetonate Lithiumacetylacetonat (Li(acac)), Natriumacetylacetonat (Na(acac)), Kaliumacetylacetonat (K(acac)) oder Calciumacetylacetonat (Ca(acac)₂) eingesetzt werden.

6. Verfahren nach einem der vorstehenden Ansprüche, **dadurch gekennzeichnet, dass** R¹ ausgewählt ist aus der Gruppe bestehend aus 1,2-Ethylen, 1,2-Propylen, 1,1-Dimethyl-1,2-ethylen, 1-Hydroxymethyl-1,2-ethylen, 2-Hydroxy-1,3-propylen, 1,3-Propylen, 1,4-Butylen, 1,5-Pentylen, 2-Methyl-1,3-propylen, 2-Ethyl-1,3-propylen, 2,2-Dimethyl-1,3-propylen und 2,2-Dimethyl-1,4-butylen.

7. Verfahren nach einem der vorstehenden Ansprüche, **dadurch gekennzeichnet, dass** R² ausgewählt ist aus der Gruppe bestehend aus 1,2-Ethylen, 1,2-Propylen, 1,1-Dimethyl-1,2-ethylen, 1-Hydroxy-methyl-1,2-ethylen, 2-Hydroxy-1,3-propylen, 1,3-Propylen, 1,4-Butylen, 1,6-Hexylen, 2-Methyl-1,3-propylen, 2-Ethyl-1,3-propylen, 2,2-Dimethyl-1,3-propylen und 2,2-Dimethyl-1,4-butylen, 1,2-Cyclopentylen, 1,3-Cyclopentylen, 1,2-Cyclohexylen, 1,3-Cyclohexylen, ortho-Phenylen, 3-Oxa-1,5-pentylen, 3,6-Dioxa-1,8-octylen und 3,6,8-Trioxa-1,8,11-undecylen.

8. Verfahren nach einem der vorstehenden Ansprüche, **dadurch gekennzeichnet, dass** (L) ausgewählt ist aus der Gruppe bestehend aus N-(2-Hydroxyethyl)-pyrrolidon, N-(2-Hydroxypropyl)-pyrrolidon, N-(2'-(2-Hydroxyethoxy)-ethyl)-pyrrolidon, N-(2-Hydroxyethyl)-caprolactam, N-(2-Hydroxypropyl)-caprolactam und N-(2'-(2-Hydroxyethoxy)-ethyl)-caprolactam.

9. Verfahren nach einem der vorstehenden Ansprüche, **dadurch gekennzeichnet, dass** während der Reaktion kontinuierlich ein sauerstoffhaltiges Gas in das Reaktionsgemisch eingeleitet wird.

## Claims

1. A process for preparing (meth)acrylic esters (F) of N-hydroxyalkylated lactams, in which cyclic N-hydroxyalkylated lactams (L) 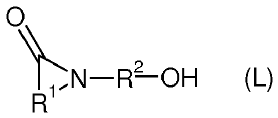 in which
R¹ is C₁-C₅-alkylene, or C₂-C₂₀-alkylene interrupted by one or more oxygen and/or sulfur atoms and/or one or more substituted or unsubstituted imino groups and/or by one or more cycloalkyl, -(CO)-, -O(CO)O-, -(NH)(CO)O-, -O(CO)(NH)-, -O(CO)- or -(CO)O- groups, where the radicals mentioned may each be substituted by aryl, alkyl, aryloxy, alkyloxy, heteroatoms and/or heterocycles,
with the proviso that R¹ must not have any atom other than a carbon atom directly adjacent to the lactam carbonyl group,
R² is C₁-C₂₀-alkylene, C₅-C₁₂-cycloalkylene, C₆-C₁₂-arylene, or C₂-C₂₀-alkylene interrupted by one or more oxygen and/or sulfur atoms and/or one or more substituted or unsubstituted imino groups and/or by one or more cycloalkyl, -(CO)-, -O(CO)O-, -(NH)(CO)O-, -O(CO)(NH)-, -O(CO) - or - (CO)O- groups, where the radicals mentioned may each be substituted by aryl, alkyl, aryloxy, alkyloxy, heteroatoms and/or heterocycles, or
R²-OH is a group of the formula -[Xᵢ]ₖ-H,
k is from 1 to 50 and
Xᵢ, for each i = 1 to k, may each independently be selected from the group of -CH₂-CH₂-O-, -CH₂-CH(CH₃)-O-, -CH(CH₃)-CH₂-O-, -CH₂-C(CH₃)2-O-, - C(CH₃)₂-CH₂-O-, -CH₂-CH₂-CH₂-O-, -CH₂-CH₂-CH₂-CH₂-O-, -CH₂-CHVin-O-, -CHVin-CH₂-O-, -CH₂-CHPh-O- and - CHPh-CH₂-O-, in which Ph is phenyl and Vin is vinyl,
in the presence of at least one catalyst (K) selected from the group consisting of alkali metal hydroxides, alkaline earth metal hydroxides and metal acetylacetonates and of a polymerization inhibitor, is esterified with (meth)acrylic acid (S) or transesterified with at least one (meth)acrylic ester (D).

2. The process according to claim 1, wherein the alkali metal hydroxides are selected from the group consisting of lithium hydroxide, sodium hydroxide and potassium hydroxide, and the alkaline earth metal hydroxides are selected from the group consisting of magnesium hydroxide and calcium hydroxide.

3. The process according to either of claims 1 and 2, wherein the catalysts (K) used are alkali metal hydroxides selected from the group consisting of lithium hydroxide, sodium hydroxide and potassium hydroxide.

4. The process according to claim 1, wherein the catalysts (K) used are metal acetylacetonates of the general formula Mₙ(C₅H₇O₂)ₙ or Mₙ(acac)ₙ in which M is selected from the group of the transition metals, alkali metals, alkaline earth metals and aluminum.

5. The process according to claim 1 or 4, wherein the metal acetylacetonates used are lithium acetylacetonate (Li(acac)), sodium acetylacetonate (Na(acac)), potassium acetylacetonate (K(acac)) or calcium acetylacetonate (Ca(acac)₂).

6. The process according to any of the preceding claims, wherein R¹ is selected from the group consisting of 1,2-ethylene, 1,2-propylene, 1,1-dimethyl-1,2-ethylene, 1-hydroxymethyl-1,2-ethylene, 2-hydroxy-1,3-propylene, 1,3-propylene, 1,4-butylene, 1,5-pentylene, 2-methyl-l,3-propylene, 2-ethyl-1,3-propylene, 2,2-dimethyl-1,3-propylene and 2,2-dimethyl-1,4-butylene.

7. The process according to any of the preceding claims, wherein R² is selected from the group consisting of 1,2-ethylene, 1,2-propylene, 1,1-dimethyl-1,2-ethylene, 1-hydroxymethyl-1,2-ethylene, 2-hydroxy-1,3-propylene, 1,3-propylene, 1,4-butylene, 1,6-hexylene, 2-methyl-1,3-propylene, 2-ethyl-1,3-propylene, 2,2-dimethyl-1,3-propylene and 2,2-dimethyl-1,4-butylene, 1,2-cyclopentylene, 1,3-cyclopentylene, 1,2-cyclohexylene,1,3-cyclohexylene, ortho-phenylene, 3-oxa-1,5-pentylene, 3,6-dioxa-1,8-octylene and 3,6,8-trioxa-1,8,11-undecylene.

8. The process according to any of the preceding claims, wherein (L) is selected from the group consisting of N-(2-hydroxyethyl)pyrrolidone, N-(2-hydroxypropyl)pyrrolidone, N-(2'-(2-hydroxyethoxy)ethyl)pyrrolidone, N-(2-hydroxyethyl)caprolactam, N-(2-hydroxypropyl)caprolactam and N-(2'-(2-hydroxyethoxy)ethyl)caprolactam.

9. The process according to any of the preceding claims, wherein an oxygenous gas is introduced continuously into the reaction mixture during the reaction.

## Revendications

1. Procédé de préparation d'esters de l'acide (méth)acrylique (F) de lactames N-hydroxyalkylés, dans lequel des lactames cycliques, N-hydroxyalkylés (L), 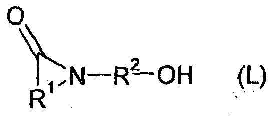 dans laquelle
R₁ signifie C₁-C₅-alkylène ; ou C₂-C₂₀-alkylène interrompu par un ou plusieurs atomes d'oxygène et/ou de soufre et/ou par un ou plusieurs groupes imino substitués ou non substitués et/ou par un ou plusieurs groupes cycloalkyle, -(CO)-, -O(CO)O-, -(NH)(CO)O-, -O(CO)(NH) -, -O(CO)- ou -(CO)O-, les radicaux mentionnés pouvant être à chaque fois substitués par aryle, alkyle, aryloxy, alkyloxy, des hétéroatomes et/ou des hétérocycles ou à condition que R¹ ne puisse pas présenter d'autre atome qu'un atome de carbone directement adjacent au groupe carbonyle du lactame,
R₂ signifie C₁-C₂₀-alkylène, C₅-C₁₂-cycloalkylène, C₆-C₁₂-arylène ; ou C₂-C₂₀-alkylène interrompu par un ou plusieurs atomes d'oxygène et/ou de soufre et/ou par un ou plusieurs groupes imino substitués ou non substitués et/ou par un ou plusieurs groupes cycloalkyle, -(CO)-, -O(CO)O-, -(NH)(CO)O-, -O(CO)(NH)-, -O(CO)- ou -(CO)O-, les radicaux mentionnés pouvant être à chaque fois substitués par aryle, alkyle, aryloxy, alkyloxy, des hétéroatomes et/ou des hétérocycles ou R₂-OHreprésente un groupe de formule -[Xᵢ]ₖ-H,
k vaut un nombre de 1 à 50 et
Xᵢ pour chaque i = 1 à k, peut être choisi indépendamment les uns des autres dans le groupe formé par -CH₂-CH₂-O-, -CH₂-CH(CH₃)-O-, -CH(CH₃)- CH₂-O-, -CH₂-C(CH₃)₂-O-, -C(CH₃)₂-CH₂-O-, -CH₂-CH₂-CH₂-O-, -CH₂-CH₂-CH₂-CH₂-O-, -CH₂-CHVin-O-, -CHVin-CH₂-O-, -CH₂-CHPh-O- et -CHPh-CH₂-O-, où Ph représente phényle et Vin représente vinyle,
en présence d'au moins un catalyseur (K) choisi dans le groupe constitué par les hydroxydes de métal alcalin, les hydroxydes de métal alcalino-terreux et les acétylacétonates métalliques et d'un inhibiteur de polymérisation, sont estérifiés avec de l'acide (méth)acrylique (S) ou transestérifiés avec au moins un ester de l'acide (méth)acrylique (D).

2. Procédé selon la revendication 1, **caractérisé en ce que** les hydroxydes de métal alcalin sont choisis dans le groupe constitué par l'hydroxyde de lithium, hydroxyde de sodium et l'hydroxyde de potassium et les hydroxydes de métal alcalino-terreux sont choisis dans le groupe constitué par l'hydroxyde de magnésium et l'hydroxyde de calcium.

3. Procédé selon l'une quelconque des revendications 1 ou 2, **caractérisé en ce qu'**on utilise, comme catalyseur (K), des hydroxydes de métal alcalin, choisis dans le groupe constitué par l'hydroxyde de lithium, hydroxyde de sodium et l'hydroxyde de potassium.

4. Procédé selon la revendication 1, **caractérisé en ce qu'**on utilise, comme catalyseurs (K), des acétylacétonates métalliques de formules générales Mₙ(C₅H₇O₂)ₙ ou Mₙ(acac)ₙ, dans lesquelles M est choisi dans le groupe formé par les métaux de transition, les métaux alcalins, les métaux alcalino-terreux et l'aluminium.

5. Procédé selon la revendication 1 ou 4, **caractérisé en ce qu'**on utilise, comme acétylacétonates métalliques, l'acétylacétonate de lithium (Li(acac)), l'acétylacétonate de sodium (Na(acac)), l'acétylacétonate de potassium (K(acac)) ou l'acétylacétonate de calcium (Ca(acac)₂).

6. Procédé selon l'une quelconque des revendications précédentes, **caractérisé en ce que** R¹ est choisi dans le groupe constitué par 1,2-éthylène, 1,2-propylène, 1,1-diméthyl-1,2-éthylène, 1-hydroxyméthyl-1,2-éthylène, 2-hydroxy-1,3-propylène, 1,3-propylène, 1,4-butylène, 1,5-pentylène, 2-méthyl-1,3-propylène, 2-éthyl-1,3-propylène, 2,2-diméthyl-1,3-propylène et 2,2-diméthyl-1,4-butylène.

7. Procédé selon l'une quelconque des revendications précédentes, **caractérisé en ce que** R² est choisi dans le groupe constitué par 1,2-éthylène, 1,2-propylène, 1,1-diméthyl-1,2-éthylène, 1-hydroxyméthyl-1,2-éthylène, 2-hydroxy-1,3-propylène, 1,3-propylène, 1,4-butylène, 1,6-hexylène, 2-méthyl-1,3-propylène, 2-éthyl-1,3-propylène, 2,2-diméthyl-1,3-propylène et 2,2-diméthyl-1,4-butylène, 1,2-cyclopentylène, 1,3-cyclopentylène, 1,2-cyclohexylène, 1,3-cyclohexylène, ortho-phénylène, 3-oxa-1,5-pentylène, 3,6-dioxa-1,8-octylène et 3,6,8-trioxa-1,8,11-undécylène.

8. Procédé selon l'une quelconque des revendications précédentes, **caractérisé en ce que** (L) est choisi dans le groupe constitué par N-(2-hydroxyéthyl)-pyrrolidone, N-(2-hydroxypropyl)-pyrrolidone, N-(2'-(2-hydroxyéthoxy)-éthyl)-pyrrolidone, N-(2-hydroxyéthyl)-caprolactame, N-(2-hydroxypropyl)-caprolactame et N-(2'-(2-hydroxyéthoxy)-éthyl)-caprolactame.

9. Procédé selon l'une quelconque des revendications précédentes, **caractérisé en ce qu'**un gaz contenant de l'oxygène est introduit en continu pendant la réaction dans le mélange réactionnel.
